Europäisches Patentamt

European Patent Office

Office européen des brevets

(19)

(11) Publication number: **0 150 572**
A1

# (12) EUROPEAN PATENT APPLICATION

(21) Application number: 84307490.7

(22) Date of filing: 31.10.84

(51) Int. Cl.⁴: **C 12 N 15/00,** C 12 P 21/02, C 07 K 7/10, A 61 K 39/00 // C12R1:19

(30) Priority: 01.11.83 US 547484
23.10.84 US 662779

(43) Date of publication of application: 07.08.85
Bulletin 85/32

(84) Designated Contracting States: AT BE CH DE FR GB IT LI LU NL SE

(71) Applicant: Amgen, 1900 Oak Terrace Lane, Thousand Oaks California 91320 (US)

(72) Inventor: Banks, Allen R., 2886 Middlefork Road, Boulder Colorado 80302 (US)
Inventor: Hare, David L., 7481 Mt. Meeker Road, Longmont Colorado 80501 (US)

(74) Representative: Brown, John David et al, FORRESTER & BOEHMERT Widenmayerstrasse 4/I, D-8000 München 22 (DE)

(54) Microbial expression of type 1 transforming growth factor, polypeptide analogs thereof and hybrid EGF/TGF polypeptides.

(57) Disclosed is the manufacture of DNA sequences comprising structural genes coding for (1) a polypeptide having the amino acid sequence and properties of Type I Transforming Growth Factor; (2) polypeptide analogs of Type I Transforming Growth Factor which differ from the naturally-occurring forms in terms of the identity and/or location of one or more amino acids, e.g., [Phe$^{15}$] rTGF, and [Asp$^7$] rTGF; and (3) hybrid polypeptides including, e.g, part or all of the Type I TGF amino acid residue sequence and part or all of the EGF amino acid residue sequence.

"MICROBIAL EXPRESSION OF TYPE I TRANSFORMING GROWTH
FACTOR, POLYPEPTIDE ANALOGS THEREOF AND HYBRID EGF/TGF
POLYPEPTIDES"

## BACKGROUND

The present invention relates generally to the
manipulation of genetic materials and, more particularly,
to the manufacture of specific DNA sequences useful in
recombinant procedures to secure the microbial expression
of Transforming Growth Factor, of polypeptide analogs
thereof, and of hybrid polypeptides comprising portions
of the sequence of amino acid residues of Transforming
Growth Factor.

Ordinarily, normal mammalian cells (including
cells explanted from tissue or cell lines) only grow in
monolayers and will not grow in soft agar because the
cells require a solid support to which they can attach.
However, the transformed phenotype, i.e., loss of density
dependent inhibition of cell growth in monolayer culture,
overgrowth in monolayer culture, characteristic change
in cellular morphology and acquisition of anchorage-
independent growth, can be conferred on normal mammalian cells by one or more members of a family of mammalian polypeptide growth factors, Transforming Growth Factors (TGFs).

Polypeptides with the properties of TGFs have been secreted by and partially purified from tumor cells of different origins, e.g., rat embryo fibroblasts transformed by murine leukemia virus [Twardzik, D.R., et al., Science, 216, 894-897 (1982)]; cells of the human metastatic melanoma cell line A2058 [Marquardt, H., et al., J.Biol.Chem., 257, 5220-5225 (1982)]; and, Moloney murine sarcoma virus-infected mouse 3T3 cells [DeLarco, J., et al., J.Biol.Chem., 255, 3685-3690 (1980)]. Intracellular forms of TGFs have also been detected in tumor cells in culture and in vivo [Roberts, A., et al., PNAS (USA), 77, 3494-3498 (1980)].

TGFs have been classified into several categories on the basis of their biological and physical properties. The only category of TGF which has been characterized to an appreciable degree is referred to as Type I TGF (alternately TGF-α, eTGF, or EGF-like TGF). In addition to promoting growth of tumor cells, having the ability to reversibly induce a transformed phenotype in normal fibroblasts and epithelial cells when grown in tissue culture, Type I TGF competes with epidermal growth factor (EGF) for binding to cellular EGF receptors. [Marquardt, H., et al., PNAS USA, 80: 4684-4688 (1983)].

Type I TGF is functionally related to epidermal growth factor ("EGF", "urogastrone") and shares several common properties. It is approximately the same size as EGF, having about fifty amino acids and an estimated molecular weight of 7,400 daltons determined by SDS polyacrylamide gel. Like EGF, Type I TGF has six cysteines in positions analogous to those in EGF which form three disulfide bridges. [See, Marquardt, J.Biol.Chem., supra, at 5224]. Unlike EGF, however, Type

I TGF is found in cell lines with the transformed phenotype, while EGF is found only in non-transformed cell types. Type I TGF expression, unlike that of EGF, appears to be correlated with the expression of an active oncogene.

At the filing date (November 1, 1983) of applicants' above-identified parent application, amino acid sequences for Type I TGF isolated from cell lines established from solid tumors in mice, rats and humans were only partially identified in the literature. Portions of the polypeptides for which sequences were published were determined to be nearly identical, with all identified residues in mouse and rat Type I TGF being identical through amino acid number 40. [Marquardt, et al., PNAS USA, supra at 4686].

Until recently, the human Type I TGF sequence was incomplete (at least two variations from mouse and rat Type I TGF within the first 34 amino acids were known and four other residues were only tentatively identified; the sequence beyond position number 34 was unknown). The complete human Type I TGF sequence was disclosed by Derynk, et al., Cell, 38: 287-297 (August 1984), and is set out below.

```
    1              5                 10
Val Val Ser His Phe Asn Asp Cys Pro Asp Ser His Thr

         15                20                 25
Gln Phe Cys Phe His Gly Thr Cys Arg Phe Leu Val Gln

             30                35
Glu Asp Lys Pro Ala Cys Val Cys His Ser Gly Tyr Val

    40                45                  50
Gly Ala Arg Cys Glu His Ala Asp Leu Leu Ala
```

A partial amino acid sequence for rat Type I TGF was reported by Marquardt, et al., PNAS USA, 80: 4684-4688 (1983), which provided the sequence up to amino acid residue 43, with no residues identified at positions 26, 30, 36, 40 and 42. L.Hood and his co-investigators at the California Institute of Technology reported the identity of the amino acid residues at 26, 30, 36, 40 and 42 and generation of the remaining sequence from amino acid 44 to 50 in September 1983. The complete rat Type I TGF sequence was thereafter presented during the Eleventh Conference on Cell Proliferation and Cancer, entitled "The Cancer Cell", held at Cold Spring Harbor Laboratories, Cold Spring Harbor, New York, on September 8-13, 1983, by George J. Todaro in the "Growth Factors" segment of the conference. The more recent publication by Marquardt, et al., Science, 223: 1079-1082 (1984) sets out the complete sequence below:

```
    1               5                   10
Val Val Ser His Phe Asn Lys Cys Pro Asp Ser His Thr

        15              20                  25
Gln Tyr Cys Phe His Gly Thr Cys Arg Phe Leu Val Gln

            30                  35
Glu Glu Lys Pro Ala Cys Val Cys His Ser Gly Tyr Val

    40                  45                  50
Gly Val Arg Cys Glu His Ala Asp Leu Leu Ala
```

If available in large quantities in biologically active form, Type I TGF of the sequences noted above (as well as polypeptide analogs thereof) would have a number of clinical and research uses. Type I TGF could be employed to raise antibodies, useful in diagnostic tests, e.g., RIAs, to detect or characterize the presence of malignant tumors which secrete Type I TGF, or to potentially aid in the immunization of animals against establishment or progression of such tumors. The amino acid sequence for rat Type I TGF is sufficiently homologous to the human Type I TGF sequence (i.e., 92% homology) that antibodies to rat Type I TGF are expected to cross-react with human Type I TGF. Thus, human tissue, blood or urine could be screened for the presence of malignant tumors employing antibodies to rat Type I TGF. Similarly, synthetic production of large quantities of Type I TGF could permit the development of a general screen for the detection of subclinical tumors and a method for monitoring treatment efficacy in patients undergoing treatment for cancer.

Further, when employed as an additive to cell culture media Type I TGF has potential utility in promoting growth of refractive cell types and may potentially have the capacity to permit growth of large cell cultures in vitro for a variety of medical or research purposes. As an example, one potential use for Type I TGF and polypeptide analogs thereof could be to replace EGF as a constituent in media designed to stimulate growth of large cultures of epithelial cells for skin grafts and the like [See, Tsao, M., et al., J.Cell.Phys., 110: 219-229 (1982)].

Additionally, tests are currently ongoing to find ways to take advantage of the common properties of EGF and Type I TGF to maximize their therapeutic potential, i.e., to improve healing of severe wounds, and the like. [See, e.g., Chemical Week, Sept. 28, 1983, pages 40-48].

Application of recombinant DNA techniques to production of human EGF has been the subject of substantial investigation by applicants. The manufacture of genes coding for microbial expression of EGF and EGF analogs is described in their co-owned, co-pending U.S. Patent Application Serial No. 486,091, entitled "The Manufacture and Expression of Genes Coding for Urogastrone and Polypeptide Analogs Thereof", filed April 25, 1983; and the disclosures of said application are specifically incorporated by reference herein. See also, European Patent Application No. 0 046 039 published February 17, 1982.

Thus far Type I TGF has been isolated from tumors of a variety of origins in microgram amounts by costly and time-consuming chemical procedures including fractionation, gel permeation chromatography and reverse phase high pressure liquid chromatography. The first public report of the use of recombinant DNA techniques for the manufacture, cloning and expression of structural genes for Type I TGF occurred after the filing date of applicants' parent U.S. Application Serial No. 547,484 in Derynk, et al., supra. No report of use of such techniques to create polypeptide analogs thereof has occurred to date. Nor have there been any published reports of attempts to generate "composite" or "fused" or "hybrid" polypeptides possessing, in part, the biochemical and immunological properties of EGF and, in part, the biochemical and immunological properties of Type I TGF.

There thus continues to be a need in the art for fully operative methods and materials suitable for use in the large-scale production of pure, biologically active Type I TGF, polypeptide analogs thereof, and hybrid polypeptides comprising portions of the amino acid residue sequences of EGF and Type I TGF.

## BRIEF SUMMARY

Provided by the present invention are manufactured genes capable of directing synthesis, in a selected host microorganism, of Type I Transforming Growth Factor. In preferred forms of manufactured genes of the invention, the base sequence codes for a polypeptide duplicative of either human or rat species Type I TGF ("hTGF" and "rTGF") and includes one or more codons selected from among alternative codons specifying the same amino acid on the basis of preferential expression characteristics for the codon in a projected host microorganism, e.g.,

E.coli. Other preferred forms of manufactured genes include those wherein there is provided a base codon specifying an additional amino acid residue in the polypeptide encoded which facilitates the direct expression in E.coli organisms (e.g., an initial Met residue).

In still other preferred forms of manufactured genes, the sequence of base codons specifying the desired polypeptide is preceded by and/or followed by and/or includes one or more sequences of bases facilitating formation of expression vectors or generation of new structural genes for polypeptide analogs, i.e., sequences of bases providing for termination of transcription, or for promoter/regulator regions, and/or sequences of bases providing for selected restriction endonuclease cleavage sites on one or both ends of the structural gene or at intermediate positions therein. Preferably sequences at one or both ends of the structural gene code for restriction endonuclease enzyme recognition sites which occur only once in the vector in which the gene is to be inserted. Intermediate sequences in the gene desirably code for restriction endonuclease enzyme recognition sites unique to the gene itself as well as not present in any vector construction.

Also provided by the present invention are manufactured genes capable of directing the microbial expression of Type I Transforming Growth Factor polypeptide analogs which differ from Type I TGF in terms of the identity and/or location of one or more amino acid residues. Specific polypeptide analogs according to the invention include, e.g., $[Asp^7]$ rTGF; $[Phe^{15}]$ rTGF; $[Ala^{41}]$ rTGF; and $[Asp^{28}]$ rTGF, which analogs more closely approximate the human Type I TGF sequence.

As a further aspect of the present invention, there are provided genes comprising manufactured Type I TGF genes and gene fragments according to the invention fused to a second gene or gene fragment which itself is capable of directing synthesis of a second biologically active polypeptide (e.g., urogastrone, epidermal growth factor, "EGF") in a manner permitting the microbial expression of a fused or hybrid polypeptide including a part or all of Type I Transforming Growth Factor polypeptide or polypeptide analog and a part or all of a second polypeptide such as EGF. Exemplary hybrid polypeptide products of such manufactured genes include the hybrid polypeptides designated "$rTGF_{1-32}/hEGF_{32-53}$", "$hEGF_{1-31}/rTGF_{33-50}$", "$hTGF_{1-32}/hEGF_{32-53}$", and "$hEGF_{1-31}/hTGF_{33-50}$".

In practice of the invention to generate polypeptide products, manufactured DNA sequences are inserted into viral or circular plasmid DNA vectors to form hybrid vectors and the hybrid vectors are employed to genetically transform host microorganisms such as bacteria (e.g., E.coli) or yeast cells. The genetically transformed microorganisms are thereafter grown under appropriate nutrient conditions and express the polypeptide products of the invention.

Also encompassed by the present invention is the development of antibody substances generated to the Type I TGF polypeptides described herein and to polypeptide analogs and fragments thereof.

Other aspects and advantages of the present invention will be apparent upon consideration of the following detailed description thereof.

DETAILED DESCRIPTION

As employed herein, the term "manufactured" as applied to a DNA sequence or gene shall designate a product either totally chemically synthesized by assembly of nucleotide bases or derived from the biological replication of a product thus chemically synthesized. As such, the term is exclusive of products "synthesized" by cDNA methods or genomic cloning methodologies which involve starting materials which are initially of biological origin.

The following abbreviations shall be employed herein to designate amino acids: Alanine, Ala; Arginine, Arg; Asparagine, Asn; Aspartic acid, Asp; Cysteine, Cys; Glutamine, Gln; Glutamic acid, Glu; Glycine, Gly; Histidine, His; Isoleucine, Ile; Leucine, Leu; Lysine, Lys; Methionine, Met; Phenylalanine, Phe; Proline, Pro; Serine, Ser; Threonine, Thr; Tryptophan, Trp; Tyrosine, Tyr; Valine, Val. The following abbreviations shall be employed for nucleotide bases: A for adenine; G for guanine; T for thymine; U for uracil; and C for cytosine.

For ease of understanding of the present invention, Table I below provides a tabular correlation between the 64 alternate triplet nucleotide base codons of DNA and the 20 amino acids and transcription termination ("stop") function specified thereby.

TABLE I

| FIRST POSITION | SECOND POSITION | | | | THIRD POSITION |
|---|---|---|---|---|---|
| | T | C | A | G | |
| T | Phe | Ser | Tyr | Cys | T |
| | Phe | Ser | Tyr | Cys | C |
| | Leu | Ser | Stop | Stop | A |
| | Leu | Ser | Stop | Trp | G |
| C | Leu | Pro | His | Arg | T |
| | Leu | Pro | His | Arg | C |
| | Leu | Pro | Gln | Arg | A |
| | Leu | Pro | Gln | Arg | G |
| A | Ile | Thr | Asn | Ser | T |
| | Ile | Thr | Asn | Ser | C |
| | Ile | Thr | Lys | Arg | A |
| | Met | Thr | Lys | Arg | G |
| G | Val | Ala | Asp | Gly | T |
| | Val | Ala | Asp | Gly | C |
| | Val | Ala | Glu | Gly | A |
| | Val | Ala | Glu | Gly | G |

The manufacture of structural genes according to the present invention is preferably carried out according to the procedures disclosed in the co-owned, co-pending U.S. Patent Application Serial No. 375,493, filed May 6, 1982 by Yitzhak Stabinsky, entitled "Manufacture and Expression of Structural Genes" [PCT International Publication No. WO83/04029, published November 24, 1983], which application is incorporated by reference herein for the purpose of describing the steps of the procedure.

The following examples illustrate practice of the invention in the manufacture of the DNA sequences coding for microbial expression of Type I hTGF, Type I rTGF, polypeptide analogs thereof, and Type I TGF/EGF hybrid polypeptides. Also illustrated is the construction of expression vectors for direct expression of desired polypeptides.

The following example is directed to the general procedure employed in the synthesis of oligonucleotide fragments for the manufacture of structural genes according to the invention.

EXAMPLE 1

Oligonucleotide fragments were synthesized using a four-step procedure and several intermediate washes. Polymer bound dimethoxytrityl protected nucleoside in a sintered glass funnel was first stripped of its 5'-protecting group (dimethoxytrityl) using 3% trichloroacetic acid in dichloromethane for 1-1/2 minutes. The polymer was then washed with dichloromethane and acetonitrile. The washed polymer was then rinsed with dry acetonitrile, placed under argon and treated in the condensation step as follows. 0.5 ml of a solution of 10 mg tetrazole in acetonitile was added to the reaction vessel containing polymer. Then 0.5 ml of 30 mg protected nucleoside phosphoramidite in acetonitrile was added. This reaction was agitated and allowed to react for 2 minutes. The reactants were then removed by suction and the polymer rinsed with acetonitrile. This was followed by the oxidation step wherein 1 ml of a solution containing 0.1 molar $I_2$ in 2-6-lutidine/$H_2$O/THF, 1:2:2, was reacted with the polymer bound oligonucleotide chain for 10 seconds. Following a THF rinse capping of unreacted nucleotides was done using a solution of dimethylaminopyridine (6.5 g in 100 ml THF) and acetic anhydride, 2,6-lutidine (1:1) in the proportion 4:1 for 2 minutes. This was followed by a methanol rinse and a THF rinse. Then the cycle began again with a trichloroacetic acid in $CH_2Cl_2$ treatment. The cycle was repeated until the desired oligonucleotide sequence was obtained.

The final oligonucleotide chain was treated with thiophenol, triethylamine, dioxane (1:1:2), for 45 minutes at room temperature. Then, after rinsing with dioxane, methanol and diethylether, the oligonucleotide was cleaved from the polymer with concentrated ammonia at room temperature. After decanting the solution from the polymer, the concentrated ammonia solution was heated at 60°C for 16 hours in a sealed tube.

Each oligonucleotide solution was then extracted four times with 1-butanol. The solution was loaded into a 20% polyacrylamide 7 molar urea electrophoresis gel and, after running, the appropriate product band was isolated.

The following example is directed to the manufacture of structural genes coding for Type I TGF and also illustrates the manner in which structural genes for polypeptide analogs thereof may be prepared.

## EXAMPLE 2

Fourteen specific deoxyoligonucleotides (numbered 1 through 14) were synthesized according to the procedures of Example 1. The oligonucleotides were purified by polyacrylamide gel electrophoresis and were then phosphorylated at the 5' ends using ATP and $T_4$ polynucleotide kinase in a standard reaction using one nanomole of DNA, a two fold excess of ATP and 1 unit of $T_4$ kinase in 20 µl of buffer made with 50 mM hydroxyethylpiperazine ethane sulfonic acid, 10 mM $MgCl_2$, 10 mM dithiothreitol, pH 7.6. After reaction, the kinase was destroyed by boiling for 5 minutes. These phosphorylated oligonucleotides in the buffer were then used directly for ligation.

The oligonucleotides in 20 µl standard buffer were combined to form short duplexes. Each duplex was formed by combining two complementary sequences in equimolar amounts, boiling the mixture, then slow cooling over a 1/2 hour period to room temperature. In this way, seven duplexes were formed.

These seven duplexes were combined sequentially, annealing each set of duplexes at 37°C for 5 minutes until the final structural gene was in a single tube ready for ligation. The oligonucleotide mixture was then made 150 µmolar in ATP and treated with 84 units of $T_4$DNA ligase for 16 hours at 4°C. The fully ligated structural gene was then purified by polyacrylamide gel electrophoresis.

Table II below illustrates the finally assembled structural gene coding for direct microbial expression of a polypeptide having the primary structure of rat Type I TGF.

## TABLE II

<u>BamHI</u>

| | | -1 | 1 | 2 | 3 | 4 | 5 | 6 | 7 | 8 | 9 |
|---|---|---|---|---|---|---|---|---|---|---|---|
| | | Met | Val | Val | Ser | His | Phe | Asn | Lys | Cys | Pro |

```
               ┌─────────1─────────┐               ┌──2──
5'-G ATC CAG ATG GTA GTT TCT CAT TTC AAC AAA TGC CCG
   3'-GTC TAC CAT CAA AGA GTA AAG TTG TTT ACG GGC
   └────────────8────────────┘
```

| 10 | 11 | 12 | 13 | 14 | 15 | 16 | 17 | NcoI 18 | 19 | 20 | 21 | 22 | 23 |
|---|---|---|---|---|---|---|---|---|---|---|---|---|---|
| Asp | Ser | His | Thr | Gln | Tyr | Cys | Phe | His | Gly | Thr | Cys | Arg | Phe |

```
──────────────────────3──────────────────┐
GAC TCT CAC ACT CAG TAC TGC TTC CAT GGT ACT TGC CGT TTC
CTG AGA GTG TGA GTC ATG ACG AAG GTA CCA TGA ACG GCA AAG
──────9──────┘            └──────────10──────────┘
```

| 24 | 25 | 26 | 27 | 28 | 29 | 30 | 31 | SphI 32 | 33 | 34 | 35 | 36 | 37 |
|---|---|---|---|---|---|---|---|---|---|---|---|---|---|
| Leu | Val | Gln | Glu | Glu | Lys | Pro | Ala | Cys | Val | Cys | His | Ser | Gly |

```
────────4────────┐            ────5──────────────────
CTG GTA CAG GAA GAG AAA CCG GCA TGC GTA TGC CAT TCT GGT
GAC CAT GTC CTT CTC TTT GGC CGT ACG CAT ACG GTA AGA CCA
──────11──────┘            └────────12────────────────
```

| 38 | 39 | 40 | 41 | 42 | 43 | 44 | 45 | 46 | BglII 47 48 | 49 | 50 |
|---|---|---|---|---|---|---|---|---|---|---|---|
| Tyr | Val | Gly | Val | Arg | Cys | Glu | His | Ala | Asp Leu | Leu | Ala |

```
────────────6────────────┐               ──────7──────
TAC GTT GGC GTA CGT TGC GAA CAT GCA GAT CTG CTG GCT TAA
ATG CAA CCG CAT GCA ACG CTT GTA CGT CTA GAC GAC CGA ATT
──────┘        └──────────13──────────┘        └──────14──────
```

<u>SalI</u>

```
TAG
ATC AGC T
────────┘
```

Table III below illustrates the finally assembled structural gene coding for direct microbial expression of a polypeptide having the primary structure of human Type I TGF.

## TABLE III

<u>BamHI</u>

| | | -1 | 1 | 2 | 3 | 4 | 5 | 6 | 7 | 8 | 9 |
|---|---|---|---|---|---|---|---|---|---|---|---|
| | | Met | Val | Val | Ser | His | Phe | Asn | Asp | Cys | Pro |

```
               ┌─────────1─────────┐               ┌──2──
5'-G ATC CAG ATG GTA GTT TCT CAT TTC AAC GAC TGC CCG
   3'-GTC TAC CAT CAA AGA GTA AAG TTG CTG ACG GGC
   └────────────8────────────┘        └─
```

TABLE III (continued)

|  | 10 | 11 | 12 | 13 | 14 | 15 | 16 | 17 | NcoI 18 | 19 | 20 | 21 | 22 | 23 |
|---|---|---|---|---|---|---|---|---|---|---|---|---|---|---|
|  | Asp | Ser | His | Thr | Gln | Phe | Cys | Phe | His | Gly | Thr | Cys | Arg | Phe |
|  |  |  |  |  |  |  |  | —————3————— |  |  |  |  |  |  |
|  | GAC | TCT | CAC | ACT | CAG | TTC | TGC | TTC | CAT | GGT | ACT | TGC | CGT | TTC |
|  | CTG | AGA | GTG | TGA | GTC | AAG | ACG | AAG | GTA | CCA | TGA | ACG | GCA | AAG |
|  | ——9—— |  |  |  |  |  |  | ————10———— |  |  |  |  |  |  |

|  | 24 | 25 | 26 | 27 | 28 | 29 | 30 | 31 | SphI 32 | 33 | 34 | 35 | 36 | 37 |
|---|---|---|---|---|---|---|---|---|---|---|---|---|---|---|
|  | Leu | Val | Gln | Glu | Asp | Lys | Pro | Ala | Cys | Val | Cys | His | Ser | Gly |
|  |  | ——4—— |  |  |  |  |  | ——5—— |  |  |  |  |  |  |
|  | CTG | GTA | CAG | GAA | GAC | AAA | CCG | GCA | TGC | GTA | TGC | CAT | TCT | GGT |
|  | GAC | CAT | GTC | CTT | CTG | TTT | GGC | CGT | ACG | CAT | ACG | GTA | AGA | CCA |
|  |  | ——11—— |  |  |  |  |  | ————12———— |  |  |  |  |  |  |

|  | 38 | 39 | 40 | 41 | 42 | 43 | 44 | 45 | 46 | BglII 47 48 | 49 | 50 |
|---|---|---|---|---|---|---|---|---|---|---|---|---|
|  | Tyr | Val | Gly | Ala | Arg | Cys | Glu | His | Ala | Asp Leu | Leu | Ala |
|  |  |  | ——6—— |  |  |  |  |  |  | ——7—— |  |  |
|  | TAC | GTT | GGC | GCA | CGT | TGC | GAA | CAT | GCA | GAT CTG | CTG | GCT TAA |
|  | ATG | CAA | CCG | CGT | GCA | ACG | CTT | GTA | CGT | CTA GAC | GAC | CGA ATT |
|  |  |  |  |  |  |  | ————13———— |  |  |  | ——14—— |  |

SalI

```
TAG
ATC AGC T
```

Included in each Table is the numbered sequence of amino acid residues specified when the DNA sequence is suitably inserted within a microbial expression vector. Each polypeptide sequence is thus seen to comprise the series of fifty amino acids of naturally-occurring rat and human Type I TGF together with an initial methionine residue (at position -1).

In the Tables, codons specifying each amino acid are aligned below the designation of the residue specified. Bracketed regions in the DNA sequence indicate the fourteen separate oligonucleotides initially formed and also designate the intermediate duplexes (e.g., 1 and 2, 3 and 4, etc.). Wherever consistent with the procedures for construction of the manufactured gene, codon usage is selected on the basis of "preferences" of the projected expression host (e.g., E.coli) according to the data set out in Grantham, et al., Nucleic Acids Research, 8, pages r49-r62 (1980); Grantham, et al., Nucleic Acids Research, 8, pages 1893-1912 (1980); and Grantham, et al., Nucleic Acids Research, 9, pages r43-r74 (1981). See, also, Bennetzen, et al., J.Biol.Chem., 257, pp. 3026-3031 (1982); and Grosjean, et al., Gene, 18, pp. 199-209 (1982).

The codons employed in generating the Type I TGF structural genes of Tables II and III are among those enabling maximal expression in E.coli. Numerous changes may be made to facilitate expression and/or facilitate manipulations involved in generating structural genes coding for Type I TGF analogs. As an example, by employing alternative codons, it was possible to introduce two unique, internal restriction sites at the first third and last third of the genes (i.e., NcoI and SphI). This permits each gene to be altered or spliced where desired to adapt to further species specific changes in the amino acid sequence. The BglII recognition site prior to the termination codons permits additional sequences to be added where desired. Codon changes may also be employed to alter secondary structure present in the manufactured structural gene.

At that end of each gene which specifies the amino terminal residues of the polypeptide to be expressed (Met$^{-1}$, Val$^{1}$, etc.) there are provided bases forming the sticky end of a selected restriction endonuclease enzyme recognition site (here, illustratively, a BamHI sticky end). At the end of each gene which specifies the carboxy terminal residue of the polypeptide (Ala$^{50}$) there are provided translation stop codons (e.g., TAA, TAG) and bases forming a sticky end of a similarly selected restriction endonuclease recognition site (here, illustratively, a SalI sticky end). It will be understood that one or more entire, duplex endonuclease recognition sites

0150572

may be provided at each end which can be enzyme treated to form a selected sticky end in the course of insertion into a vector.

A "leader" DNA sequence may be provided at the 5' end of the coding strand to provide alternate restriction endonuclease recognition sites, permitting the Type I TGF sequence to be inserted behind selected promoter/operators. As an example, the leader DNA sequence set out below facilitates insertion of a Type I TGF gene adjacent to a synthetic T5 early promoter replica DNA sequence (specifically, the T5A-lac promoter/operator sequence, disclosed in co-owned, co-pending U.S. Patent Application Serial No. 521,959 by Banks, et al., filed August 10, 1983 and entitled "Enhancement of Microbial Expression of Polypeptides", which is incorporated by reference herein), or the $\lambda P_L$ promoter [see, e.g., Shimatake, et al., Nature, 292: 128-132 (1981)] or the commonly used trp promoter for direct expression:

```
                   -8  -7  -6  -5  -4  -3  -2  -1
                   Met Lys Lys Tyr Trp Ile Gln Met
5'-GAA TTC TCT AGA ATG AAG AAA TAT TGG ATC CAG ATG-3'
    EcoRI   XbaI                        BamHI
```

The XbaI site permits the polypeptide coding sequence to be inserted 3' to a trp promoter for production of a polypeptide having a short "pro" polypeptide sequence cleavable at $\mathrm{Met}^{-1}$ with cyanogen bromide. The leader DNA sequence with its EcoRI recognition site also enables the construction of a fusion peptide with the β-galactosidase enzyme, which also possesses an EcoRI recognition site intermediate in the gene coding sequence. A leader DNA sequence as above may be manufactured at the same time as the structural gene or added by insertion of the structural gene into a plasmid already incorporating the sequence.

Novel Type I TGF analog genes coding for microbial expression of polypeptide sequences differing

0150572

- 17 -

from the natural Type I TGF sequence may also be prepared
by altering the base sequences of selected oligonucleotides and repeating the ligation step to obtain the new
genes. For example, by altering oligonucleotide numbers
2 and 9 in rat Type I TGF, the codon specifying lysine
at residue 7 may be changed to one specifying aspartic
acid, which is the residue at position 7 of human Type
I TGF. The codon modification in oligonucleotide number
2 would be from 5'-AAA-3' to 5'-GAT-3' or 5'-GAC-3'.
The modification in rat Type I TGF oligonucleotide number
9 would be from 3'-TTT-5' to 3'-CTA-5' or 3'-CTG-5'.

Similarly by altering rat Type I TGF oligonucleotide numbers 3 and 10, and changing the tyrosine at
residue 15 to phenylalanine, the sequence becomes more
similar to the human sequence. The codon modifications
in oligonucleotide number 3 are from 5'-TAC-3' to
5'-TTC-3'. In oligonucleotide number 10, the complementary codon changes are from 3'-ATG-5' to 3'-AAG-5'.

Several codon modifications can be employed
to alter the sequence of any desired amino acid to any
other amino acid accordingly.

The following examples relate to procedures
for construction of expression vectors for use in transformation of microbial host cells to secure expression
of certain Type I TGF polypeptide products of the
invention.

EXAMPLE 3

A. Construction of Plasmid pADH15

The 166 base pair manufactured Type I rTGF gene
illustrated in Table II of Example 2 and having BamHI
and SalI sticky ends was inserted into a similarly
digested E.coli pBR322 derived cloning vector pTP which
contains the leader DNA sequence of Example 2 having

EcoRI and XbaI restriction endonuclease enzyme recognition sites located 5' to its BamHI restriction endonuclease enzyme recognition site. Resulting plasmid pADH12 contained the Type I TGF gene sequence 3' to the following DNA leader sequence already present in the original vector:

5'-GAA TTC TCT AGA ATG AAG AAA TAT TGG ATC C-3'.
    EcoRI   XbaI                        BamHI

Clones with the gene were characterized by polyacrylamide gel electrophoresis to verify the estimated molecular weight for the Type I TGF structural gene.

pADH12 was cleaved with restriction endonuclease enzymes XbaI and SalI, and the small fragment containing bases coding for the leader sequence followed by the 166 base pair Type I TGF coding sequence was inserted into a similarly-digested pBR322-derived expression plasmid pInt-γ-txB4 which contains a tryptophan synthetase (trp) promoter sequence and an XbaI site 3' to a Shine/Dalgarno sequence followed by a structural gene coding for another polypeptide. See, co-owned, co-pending U.S. Patent Application Serial No. 483,451, by Alton, et al., filed April 15, 1983 [PCT International Publication No. WO83/04053, published November 24, 1983] and entitled "The Manufacture and Expression of Large Structural Genes", which is incorporated by reference herein. The resulting plasmid, pADH15, therefore has the Type I rTGF gene placed under the control of the trp promoter. The insertion of the leader sequence and Type I rTGF gene in the correct orientation has been verified by restriction enzyme analysis and molecular weight sizing using polyacrylamide gel electrophoresis.

Vector pADH15 is employed as an expression vector in an E.coli host to generate a polypeptide product which includes a "pro" sequence of 8 amino acids as follows: $NH_2$-$Met^{-8}$-$Lys^{-7}$-$Lys^{-6}$-$Tyr^{-5}$-$Trp^{-4}$-$Ile^{-3}$-$Gln^{-2}$-$Met^{-1}$-[TGF]-COOH.

### B. Construction of Plasmid pADH96

Upon association of the manufactured Type I rTGF structural gene with a suitable promoter/operator (trp) within plasmid pADH15, further manipulations were performed in an attempt to generate an expression vector which would provide for high levels of E.coli expression of the desired protein. These manipulations involved use of a plasmid which is the subject of co-owned, co-pending, U.S. Patent Application Serial No. 521,964, by Morris, filed August 10, 1983 and entitled "DNA Plasmids". The disclosures of said application are specifically incorporated by reference herein. Briefly noted, plasmid pCFM414 (A.T.C.C. 40076), employed in the construction, includes a temperature-sensitive mutation in the copy control region. Upon transformation with this vector, host cell cultures grown at 30°C will have a low number of copies of the plasmid. The plasmid copy number increases fifty-fold (i.e., "runs away") within the host cells upon elevation of the culture temperature above 34°C. Growth at 37°C will ordinarily be lethal to the cells.

The specific manipulations involved in construction of a vector using A.T.C.C. 40076 were as follows. Plasmid pADH15 was digested with EcoRI (5' to the trp promoter) and SalI to generate an approximately 480 base pair fragment including the Type I rTGF structural gene, codons for the leader sequence and the associated trp promoter/operator. Plasmid pCFM414 was digested with EcoRI and XhoI and the large fragment was isolated. The EcoRI/SalI fragment containing the Type I TGF gene from pADH15 was then ligated with the large EcoRI/XhoI fragment of pCFM414 to generate plasmid pADH96. While this construction retained an intact EcoRI recognition site and, while ligation of the complementary sticky ends of SalI and XhoI did not restore either recognition site, a BamHI site adjacent to the XhoI site of pCFM414 was retained. It is also noteworthy that this construction resulted in the placement of the Type I rTGF structural gene 5' to transcription terminator sequence, Toop, present 3' to the XhoI site in pCFM414.

The above procedures provide for construction of pADH96 by incorporation of gene of Table II into first and second intermediate plasmids (pADH12 and pADH15 wherein the gene was first associated with a leader sequence and then with the trp promoter), prior to its insertion into pCFM414 cut with EcoRI and XhoI. A duplicate plasmid could be constructed by adding a "linker" sequence including a BamHI sticky end to the Table II gene at the terminal SalI sticky end and inserting in the correct orientation the resulting BamHI (double) sticky-ended sequence into the large fragment resulting from BamHI digestion of plasmid pADH59 disclosed in the above-noted applicants' co-pending U.S. Patent Application Serial No. 486,091, filed April 25, 1983 [PCT International Publication No. WO83/04030, published November 24, 1983], entitled "The Manufacture and Expression of Genes for Urogastrone and Polypeptide Analogs Thereof", the disclosures of which are incorporated by reference herein.

Plasmid pADH59, which is harbored in E.coli JM103 cells deposited as ATCC 39335, includes a manufactured gene coding for urogastrone, a leader sequence as described above and the trp promoter/regulator derived from pInt-γ-txB4, all inserted into pCFM414. The urogastrone gene is excisable by means of BamHI digestion, leaving as the large fragment a precise duplicate of pADH96 if it were subjected to the same enzymatic digestion. If, therefore, the Table II manufactured sequence is provided with a linker including the fol-

lowing sequence, $^{5'-TCGAG-3'}_{3'-CCTAG-5'}$, it can be inserted into the BamHI digestion product of pADH59 to yield a duplicate of pADH96.

## EXAMPLE 4

### A. Construction of Plasmid pADH123

The 166 base pair manufactured Type I hTGF gene illustrated in Table III of Example 2, having BamHI and SalI sticky ends, was amplified by insertion into a similarly cleaved M13mp9 vector, resulting in plasmid pADH120. The Type I hTGF gene was thereafter cleaved with BamHI/SalI from pADH120 and inserted into BamHI/SalI-digested pADH25. pADH25 was also previously described in the above-noted U.S. Patent Application Serial No. 486,091. Briefly put, pADH25 contained a portion of a pBR322-derived plasmid (pINT-γ-Txb4) with a manufactured XbaI site following the trp promoter/regulator sequence, a DNA "linker" sequence constructed with an internal XbaI recognition site as set out below

```
EcoRI    XbaI                          BamHI
GAA TTC TCT AGA ATG AAG AAA TAT TG
    AGA TCT TAC TTC TTT ATA ACC TAG
```

and a manufactured urogastrone sequence.

pADH25 was digested with BamHI/SalI to remove the sequence coding for urogastrone and the BamHI/SalI Type I hTGF sequence was inserted therein, thus producing expression vector pADH123. Vector pADH123 was employed as an expression vector in an E.coli host to generate a polypeptide product which includes the 8-amino acid "pro" sequence identified in Example 3A above.

### B. Construction of Plasmid pADH131

Procedures similar to those described for the enhanced expression of Type I rTGF in Example 3B above were followed for the Type I hTGF manufactured sequence. Plasmid pADH123 was digested with EcoRI (5' to the trp promoter) and SalI to generate an approximately 480 base pair fragment including the Type I hTGF structural gene, codons for the leader sequence and the associated trp promoter/operator. Plasmid pCFM414 was digested with EcoRI and XhoI and the large fragment was isolated. The EcoRI/SalI fragment containing the Type I hTGF gene from pADH123 was then ligated with the large EcoRI/XhoI fragment of pCFM414 to generate plasmid pADH131, thereby placing the Type I hTGF structural gene 5' to transcription terminator sequence, Toop, present 3' to the XhoI site in pCFM414. This construction retained an intact EcoRI recognition site but ligation of the complementary sticky ends of SalI and XhoI did not restore either recognition site. A BamHI site adjacent to the XhoI site of pCFM414 was retained.

The following example relates to microbial expression of desired Type I TGF polypeptides of the invention.


EXAMPLE 5

Expression of Type I TGF


Plasmid pADH96 (Example 3B) was transformed into E.coli strain JM103 and grown in L-broth. Clones with the correct Type I rTGF fragment were isolated and characterized by polyacrylamide electrophoresis. A product of the correct estimated size for Type I rTGF polypeptide was visualized by staining of the gel.

Plasmid pADH131 (Example 4B) was transformed into E.coli strain JM103 and grown in L-broth. Clones with the correct Type I hTGF fragment were isolated and characterized by polyacrylamide electrophoresis. A product of the correct estimated size for Type I hTGF polypeptide was visualized by staining of the gel.

- 23 -

The following example relates to the determination of biological activity of the Type I TGF polypeptides of the present invention.

### EXAMPLE 6

Prior to performance of the following three assays, the Type I rTGF and Type I hTGF polypeptides including the 8-amino acid leader expressed by pADH96 and pADH131 in E.coli (Example 4) was recovered from the host cells through the solubilization and serial reducing and oxidizing treatments described in co-owned, co-pending U.S. Patent Application Serial No. 521,908, by Banks, filed August 10, 1983 and entitled "Enhancement of Biological Activity of Microbially Expressed Polypeptides"; and the disclosures of said application are specifically incorporated by reference herein.

#### A. EGF Radioreceptor Assay

An EGF radioreceptor assay was performed according to the procedures described in Marquardt, et al., PNAS (USA), 80, supra at page 4685, employing either the Type I rTGF or Type I hTGF polypeptide of the present invention and A431 human epidermoid carcinoma cells. The Type I rTGF and Type I hTGF of the present invention were each found to be biologically active in this assay, competing with $^{125}$I-EGF for binding to the EGF receptors on the A431 cells.

#### B. Tritiated Thymidine Uptake Assay

A $^3$H-thymidine uptake assay essentially according to procedures described in Hollenberg, M., et al., J.Biol.Chem., 250, 3846-3853 (1975) was performed employing the Type I rTGF polypeptide of the present invention and human foreskin fibroblasts. The Type I rTGF of the present invention was determined to be mitogenic in this assay, and therefore biologically active.

## C. Soft Agar Colony Forming Assay

The Type I rTGF and Type I hTGF polypeptides of the present invention were also assayed for biological activity through a Soft Agar Colony Forming Assay according to procedures described in Marquardt, H., et al., J.Biol.Chem., 257, supra at page 5221, using normal rat kidney fibroblasts. Biological activity of the Type I rTGF and Type I hTGF polypeptides was confirmed by the formation of colonies on the soft agar in this assay.

## D. Antibody Development

Research efforts are presently directed to the generation of antibody substances to the polypeptides discussed and disclosed herein. More specifically, procedures for the development of monoclonal antibodies to Type I hTGF and Type I rTGF are currently ongoing. It has been preliminarily determined, however, that mice immunized with the recombinant Type I hTGF which had been reduced with dithiothreitol (which disrupts the three disulfide bonds of the molecule) produced polyvalent antibody in serum. In preliminary tests this antibody has proven capable of recognizing Type I rTGF obtained from bona fide biological sources when reduced with dithiothreitol. In addition, it recognizes recombinant dithiothreitol-reduced Type I rTGF, and its immunogen recombinant dithiothreitol-reduced Type I hTGF. The antibody, however, has not presently shown any recognition of Type I rTGF in its natural biological conformation.

It is further expected that both polyvalent and monoclonal antibody substances may be developed against polypeptide analogs of rTGF and hTGF and polypeptide fragments thereof by practice of conventional techniques known to those skilled in immunological research. [See, e.g., techniques for development of hybridoma cell lines described in Paul (ed.), Fundamental Immunology, Ch. 28, pp. 751-765, Raven Press, New York (1981) and references cited therein.] Of particular interest is the development of monoclonal antibodies to the Type I TGF polypeptides, analogs and fragments for potential use as reagents in diagnostic assays for the quantitation and detection of Type I TGF from biological sources.

The following example relates to procedures for construction of expression vectors for use in transformation of microbial host cells to secure expression of hybrid polypeptide products of the invention comprising hybrid polypeptides incorporating a portion of the amino acid sequence of rat Type I TGF and a portion of the amino acid sequence of human urogastrone (hEGF).

## EXAMPLE 7

### A. Construction of Plasmids pADH55 and pADH100

A hybrid gene coding for the first 32 amino acids at the amino terminus of Type I rTGF and the last 22 amino acids at the carboxy terminus of human EGF was constructed according to the following procedures. Plasmid pADH25, a pBR322-derived plasmid containing the trp promoter and a DNA sequence coding for an 8-amino acid "pro" sequence, followed by a gene coding for urogastrone (EGF), as disclosed in the aforementioned U.S. Patent Application Serial No. 486,091, was digested with BamHI and SphI. The coding region for the first 32 amino acids of human EGF was thereby excised from plasmid pADH25, which was then treated with bacterial alkaline phosphatase. Plasmid pADH12 (Example 3A) containing the Type I TGF structural gene was similarly digested with BamHI and SphI and the 101 base pair BamHI/SphI fragment was isolated by polyacrylamide gel electrophoresis. The isolated Type I TGF fragment from pADH12 was then mixed with the BamHI/SphI-digested fragment from plasmid pADH25 containing the trp promoter and incubated with DNA ligase to provide, upon transformation into E.coli JM103, plasmid pADH55 coding for the Type I TGF-EGF hybrid protein.

pADH55 isolated from JM103 culture was then digested with EcoRI and SalI to excise the complete trp promoter and the gene coding region providing for the first 32 amino acids of Type I TGF and last 22 amino acids of EGF. This fragment was then ligated into EcoRI/XhoI-digested pCFM414 as described for the generation of pADH96 in Example 3B above to place the promoter-gene fragment in the resulting temperature-sensitive high expression plasmid pADH100.

Because the Type I hTGF sequence similarly has the same SpHI restriction site designed into Type I rTGF, the hybrid Type I $hTGF_{1-32}/hEGF_{32-53}$ can similarly be formed and expressed by one skilled in the techniques described above.

### B. Construction of Plasmids pADC530 and pADC533

A hybrid gene coding for the first 31 amino acids at the amino terminus of human EGF and the last 18 amino acids at the carboxy terminus of Type I rTGF was constructed as described in the following manipulations. Plasmid pADH25 (Example 7A) was digested with SpHI and SalI to excise the coding region for the last 22 amino acids of EGF and thereafter the pADH25 fragment containing

the trp promoter was treated with bacterial alkaline phosphatase. Plasmid pADH12 containing the Type I TGF structural gene was similarly digested with SpHI and SalI and the SpHI/SalI fragment isolated by polyacrylamide gel electrophoresis. The isolated fragment from pADH12 was then incubated with the promoter-containing fragment from digested plasmid pADH25 and DNA ligase to provide plasmid pADC530, which codes for the EGF-Type I TGF hybrid protein upon transformation into E.coli JM103.

pADC530 isolated from JM103 culture was then digested with EcoRI and SalI to excise the complete trp promoter and gene coding region for the first 31 amino acids of EGF and last 18 amino acids of Type I TGF. This fragment was then ligated into EcoRI/XhoI-digested pCFM414 as described above, resulting in the promoter-gene fragment in the temperature-sensitive high expression plasmid pADC533.

As described above, the analogous hybrid $hEGF_{1-31}$/Type I $hTGF_{33-50}$ may also be designed and expressed employing the procedures recited above.

The following example relates to microbial expression of desired Type I TGF/EGF hybrid polypeptides of the invention.

## EXAMPLE 8
### Expression of Type I TGF/EGF Hybrids

Plasmid pADH100, transformed into E.coli strain JM103 and grown in L-broth expressed the hybrid polypeptide (designated "Type I $rTGF_{1-32}$/$hEGF_{32-53}$"), containing the first 32 amino acids of Type I TGF and the last 22 amino acids of EGF, at approximately 80 mg/OD-liter.

The hybrid polypeptide (designated "$hEGF_{1-31}$/Type I $rTGF_{33-50}$") containing the first 31 amino acids of EGF and the last 18 amino acids of Type I TGF in the

0150572

- 28 -

temperature-sensitive high expression plasmid pADC533, transformed into E.coli JM103 and grown in L-broth, was quantified at approximately 80 mg/OD-liter.

The foregoing illustrative examples are principally directed to construction of manufactured structural genes for Type I TGF and analog polypeptides and for Type I TGF hybrid polypeptides which are suited for use in direct expression and fusion product expression in E.coli host cells. Manufactured genes could readily be constructed in a manner especially well suited for direct expression in yeast cells through use of yeast preference codons. Further, the procedures of co-owned, co-pending U.S. Patent Application Serial No. 487,753, filed April 22, 1983, by Bitter and entitled "Secretion of Exogenous Polypeptides from Yeast" could be employed to secure yeast expression coupled with secretion of polypeptide products into the growth medium. In such a case it would be unnecessary to provide a methionine-specifying, translation initiating ATG codon 5' to the Type I TGF-specifying codons.

While the foregoing illustrative examples specifically relate to synthesis of hybrid polypeptides involving rat Type I TGF and human EGF fragments, it will be understood that hybrids involving the human or other species Type I TGF sequence are within the contemplation of the invention.

Further, while certain of the examples relate to hybrids comprising approximately one-third of the sequence of residues of Type I TGF or EGF and two-thirds of the sequence of residues of the alternative growth factor, it should be understood that other forms of hybrid polypeptides can be synthesized which may possess valuable composite biological activities. Analysis by the methods of Hopp and Woods, PNAS (USA), 78, 3824 (1981) reveals, for example, that naturally-occurring

Type I TGF includes three principal hydrophilic regions. These are located, respectively, at or near residues 8, 28, and 44. Additionally, preliminary studies carried out on synthetic fragments of EGF have tentatively identified the central region of the polypeptide as being highly significant to binding to EGF receptors on cells. It is thus projected that hybrid polypeptides comprising amino and carboxy terminal portions duplicating those of Type I TGF and central portions duplicating EGF may display desirable Type I TGF-like properties and yet have desirable characteristics which are equivalent to those of naturally-occurring EGF.

Products of the present invention and/or antibodies thereto may be suitably "tagged", for example radiolabelled (e.g., with $I^{125}$) conjugated with enzymes or fluorescently labelled, to provide reagent materials useful in assays and/or diagnostic test kits, for the qualitative and/or quantitative determination of the presence of such products and/or said antibodies in fluid samples. Such antibodies may be obtained from the inoculation of one or more animal species (e.g., mice, rabbit, goat, human, etc.) or from monoclonal antibody sources. Any of such reagent materials may be used alone or in combination with a suitable substrate, e.g., coated on a glass or plastic particle or bead.

Numerous modifications and variations in the practice of the invention are expected to occur to those skilled in the art upon consideration of the foregoing illustrative examples. Consequently, the invention should be considered as limited only to the extent reflected by the appended claims.

ATCC 40076 was deposited with American Type Culture Collection on 21st July, 1984. ATCC was deposited with A.T.C.C. on 14.04.03 .

The features disclosed in the foregoing description and in the following claims, may, both separately and in any combination thereof, be material for realising the invention in diverse forms thereof.

0150572

- 30 -

CLAIMS

1. A manufactured gene capable of directing the synthesis in a selected host microorganism of Type I Transforming Growth Factor polypeptide.

2. A manufactured gene according to claim 1 wherein the base sequence includes one or more codons, selected from among alternative codons specifying the same amino acid, on the basis of preferential expression characteristics of the codon in a projected host microorganism.

3. A manufactured gene according to claim 1 wherein the base sequence includes one or more codons, selected from among alternative codons specifying the same amino acid, on the basis of preferential expression characteristics of the codon in E.coli.

4. A manufactured gene according to claim 1 wherein base codons specifying Type I Transforming Growth Factor include initial and/or terminal codons respectively specifying additional initial and/or terminal amino acids in the polypeptide synthesized.

5. A manufactured gene according to claim 4 wherein said initial codons specifying additional initial amino acids are codons specifying an initial methionine residue.

6. A manufactured gene according to claim 4 wherein said initial codons specifying additional initial amino acids are codons specifying the following amino acid leader sequence:

$NH_2$-Met-Lys-Lys-Tyr-Trp-Ile-Gln-Met-COOH.

7. A manufactured gene according to claim 6 wherein said leader initial codons comprise the following sequence of bases:

5'-ATG AAG AAA TAT TGG ATC CAG ATG-3'.

8. A manufactured gene according to claim 1 wherein the base codons specifying Type I Transforming Growth Factor are preceded and/or followed by and/or includes a sequence of bases comprising a portion of a base sequence which provides a recognition site for restriction endonuclease enzyme cleavage.

9. A manufactured gene specifying rat Type I Transforming Growth Factor according to either of claims 1 or 4 or 8 wherein the base sequence comprises the following:

```
          10         20         30         40         50
          .          .          .          .          .
GATCCAGATG GTAGTTTCTC ATTTCAACAA ATGCCCGGAC TCTCACACTC
    GTCTAC CATCAAAGAG TAAAGTTGTT TACGGGCCTG AGAGTGTGAG
BamHI     60         70         80         90        100
          .          .          .          .          .
AGTACTGCTT CCATGGTACT TGCCGTTTCC TGGTACAGGA AGAGAAACCG
TCATGACGAA GGTACCATGA ACGGCAAAGG ACCATGTCCT TCTCTTTGGC
         110  NcoI   120        130        140        150
          .          .          .          .          .
GCATGCGTAT GCCATTCTGG TTACGTTGGC GTACGTTGCG AACATGCAGA
CGTACGCATA CGGTAAGACC AATGCAACCG CATGCAACGC TTGTACGTCT
SphI      160                                        BgIII
          .
TCTGCTGGCT TAATAG
AGACGACCGA ATTATCAGCT
                SalI
```

10. A manufactured gene capable of directing the synthesis in a selected host microorganism of a Type I Transforming Growth Factor polypeptide analog which differs therefrom in terms of the identity and/or location of one or more amino acids.

11. A manufactured gene according to claim 10 wherein the base sequence includes one or more codons,

0150572

- 32 -

selected from among alternative codons specifying the same amino acid, on the basis of preferential expression characteristics of the codon in a projected host micro-organism.

12. A manufactured gene according to claim 11 wherein the base sequence includes one or more codons, selected from among alternative codons specifying the same amino acid, on the basis of preferential expression characteristics of the codon in E.coli.

13. A manufactured gene according to claim 10 and specifying a rat Type I Transforming Growth Factor polypeptide analog selected from the group consisting of: [Asp$^7$] rTGF; [Phe$^{15}$] rTGF; [Asp$^{28}$] rTGF; [Ala$^{41}$] rTGF; [Met$^{-1}$] rTGF; and [Met$^{-8}$, Lys$^{-7}$, Lys$^{-6}$, Tyr$^{-5}$, Trp$^{-4}$, Ile$^{-3}$, Gln$^{-2}$, Met$^{-1}$] rTGF.

14. A manufactured gene according to claim 10 and specifying a human Type I Transforming Growth Factor polypeptide analog selected from the group consisting of: [Met$^{-1}$] hTGF and [Met$^{-8}$, Lys$^{-7}$, Lys$^{-6}$, Tyr$^{-5}$, Trp$^{-4}$, Ile$^{-3}$, Gln$^{-2}$, Met$^{-1}$] hTGF.

15. A manufactured gene according to claim 10 wherein base codons specifying a Type I Transforming Growth Factor polypeptide analog include initial and/or terminal codons respectively specifying additional initial and/or terminal amino acids in the polypeptide synthesized.

16. A manufactured gene according to claim 10 wherein said initial codons specifying additional initial amino acids are codons specifying the following amino acid leader sequence:

NH$_2$-Met-Lys-Lys-Tyr-Trp-Ile-Gln-Met-COOH.

17. A manufactured gene according to claim 16 wherein said leader initial codons specifying said amino acid pro sequence comprise the following sequence of bases:

5'-ATG AAG AAA TAT TGG ATC CAG ATG-3'.

18. A manufactured gene according to claim 10 wherein the base codons specifying Type I Transforming Growth Factor polypeptide analog are preceded and/or followed by and/or include a sequence of bases comprising a portion of a base sequence which provides a recognition site for restriction endonuclease cleavage of a DNA sequence.

19. A fusion gene comprising a manufactured gene according to either of claims 1 or 10 fused to a second gene capable of directing synthesis of a second polypeptide in a manner permitting the synthesis of a fused hybrid polypeptide including the polypeptide products coded for by the manufactured genes of either of claims 1 or 10.

20. A manufactured fusion gene capable of directing the synthesis in a selected host microorganism of a polypeptide comprising a selected portion of the amino acid sequence of Type I Transforming Growth Factor and a selected portion of the amino acid sequence of Epidermal Growth Factor.

21. A manufactured fusion gene capable of directing the synthesis in a selected host microorganism of a polypeptide comprising the first 32, amino terminal, amino acid residues of Type I Transforming Growth Factor and the last 22, carboxy terminal, amino acid residues of human Epidermal Growth Factor.

22. A manufactured fusion gene capable of directing the synthesis in a selected host microorganism of a polypeptide comprising the first 31, amino terminal, amino acid residues of human Epidermal Growth Factor and the last 18, carboxy terminal, amino acid residues of Type I Transforming Growth Factor.

23. A biologically functional DNA microorganism transformation vector including a manufactured gene according to either of claims 1 or 10.

24. A biologically functional DNA microorganism transformation vector including a fusion gene according to claim 19.

25. A biologically functional DNA microorganism transformation vector including a fusion gene according to either of claims 20, 21, or 22.

26. A vector according to claim 23 which is a circular DNA plasmid.

27. A vector according to claim 24 which is a circular DNA plasmid.

28. A vector according to claim 25 which is a circular DNA plasmid.

29. A microorganism transformed with a vector according to claim 23.

30. A microorganism transformed with a vector according to claim 24.

31. A microorganism transformed with a vector according to claim 25.

32. A process for the production of Type I Transforming Growth Factor polypeptide comprising:

growing, under appropriate nutrient conditions, microorganisms transformed with a biologically functional DNA including a manufactured gene according to either of claims 1 or 10, whereby said microorganisms express said gene and produce Type I Transforming Growth Factor polypeptide.

33. A process according to claim 32 wherein the microorganisms grown are E.coli microorganisms.

34. A polypeptide product of the expression in a microorganism of a manufactured gene according to either of claims 1 or 10.

35. A product according to claim 34 which is selected from the group consisting of: [Met$^{-1}$] rTGF; [Met$^{-1}$] hTGF; [Asp$^7$] rTGF; [Phe$^{15}$] rTGF; [Ala$^{41}$] rTGF; [Asp$^{28}$] rTGF; [Met$^{-8}$, Lys$^{-7}$, Lys$^{-6}$, Tyr$^{-5}$, Trp$^{-4}$, Ile$^{-3}$, Gln$^{-2}$, Met$^{-1}$] rTGF; and [Met$^{-8}$, Lys$^{-7}$, Lys$^{-6}$, Tyr$^{-5}$, Trp$^{-4}$, Ile$^{-3}$, Gln$^{-2}$, Met$^{-1}$] hTGF.

36. A polypeptide product of the expression in a microorganism of a manufactured gene according to claim 19.

37. A polypeptide product of the expression in a microorganism of a manufactured gene according to either of claims 20, 21, or 22.

38. A reagent material comprising a radiolabelled manufactured gene according to either of claims 1 or 10.

39. A reagent material comprising a radiolabelled polypeptide product of claim 34, 36, or 37.

40. A reagent material according to claim 38 wherein said radiolabel is $I^{125}$.

41. A reagent material according to claim 39 wherein said radiolabel is $I^{125}$.

42. A reagent material comprising a tagged antibody to a polypeptide according to claim 34, 36 or 37 coated on the surface of a plastic bead.

43. An antibody substance capable of binding to a Type I Transforming Growth Factor polypeptide or polypeptide analog or fragment thereof.

44. An antibody substance according to claim 43 which is a polyvalent antibody.

45. An antibody substance according to claim 43 which is a monoclonal antibody.

0150572

**European Patent Office**

Application number:

84307490.7

# DECLARATION PURSUANT TO RULE 28, PARAGRAPH 4,
# OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**     ATCC 40076

ATCC 39335

| | **DOCUMENTS CONSIDERED TO BE RELEVANT** | | EP 84307490.7 |
|---|---|---|---|
| Category | Citation of document with indication, where appropriate, of relevant passages | Relevant to claim | CLASSIFICATION OF THE APPLICATION (Int. Cl.4) |
| X,D, P | CELL, vol. 38, no. 1, August 1984, Cambridge, Mass., USA<br><br>R. DERYNCK et al. "Human Transforming Growth Factor-$\alpha i$ Precursor Structure and Expression in E.coli"<br>pages 287-297<br><br>* Pages 287-291 *<br><br>-- | 1-5 | C 12 N 15/00<br><br>C 12 P 21/02<br><br>C 07 K   7/10<br><br>A 61 K 39/00<br><br>//C 12 R   1:19 |
| A,D, P | SCIENCE, vol. 223, no. 4640, March 9, 1984, Washington D.C., USA<br><br>H. MARQUARDT et al. "Rat Transforming Growth Factor Type 1:<br><br>Structure and Relation to Epidermal Growth Factor"<br>pages 1079-1082<br><br>* Totality *<br><br>-- | 1,35 | |
| A,D | PROCEEDINGS OF THE NATIONAL ACADEMY OF SCIENCES OF THE UNITED STATES OF AMERICA, vol. 80, no. 15, August 1983, Baltimore, USA<br><br>H. MARQUARDT et al. "Transforming growth factors produced by retrovirus-transformed rodent fibroblasts and human melanoma cells: Amino acid sequence homology with epidermal growth factor"<br>pages 4684-4688<br><br>* Totality *<br><br>-- | 1,35 | **TECHNICAL FIELDS SEARCHED (Int. Cl.4)**<br><br>C 12 N<br><br>C 12 P<br><br>C 07 K<br><br>A 61 K |

The present search report has been drawn up for all claims

| Place of search | Date of completion of the search | Examiner |
|---|---|---|
| VIENNA | 21-01-1985 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| | DOCUMENTS CONSIDERED TO BE RELEVANT | | EP 84307490.7 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | EP – A1 – 0 046 039 (G.D. SEARLE & CO.)<br><br>* Claims; fig. 1 *<br><br>–– | 1,23, 32,34, 35 | |
| A | DE – A1 – 2 540 544 (IMPERIAL CHEMICAL INDUSTRIES LTD.)<br><br>* Claim 2 *<br><br>–– | 35 | |
| A | DE – A1 – 2 540 545 (IMPERIAL CHEMICAL INDUSTRIES LTD.)<br><br>* Claim 2 *<br><br>–––– | 35 | |
| | | | TECHNICAL FIELDS SEARCHED (Int. Cl.4) |

The present search report has been drawn up for all claims

| Place of search<br>VIENNA | Date of completion of the search<br>21-01-1985 | Examiner<br>WOLF |
|---|---|---|

EPO Form 1503 03 82